**Europäisches Patentamt**

**European Patent Office**

**Office européen des brevets**

(19)

(11) Veröffentlichungsnummer: **0 347 606**
**A1**

(12) **EUROPÄISCHE PATENTANMELDUNG**

(21) Anmeldenummer: 89109360.1

(51) Int. Cl.⁴: **A61B 19/04**

(22) Anmeldetag: 24.05.89

(30) Priorität: 22.06.88 CH 2392/88

(43) Veröffentlichungstag der Anmeldung:
27.12.89 Patentblatt 89/52

(84) Benannte Vertragsstaaten:
AT BE DE FR GB IT LU NL SE

(71) Anmelder: **Herzog, Kurt**
**Sennrüti 7**
**CH-9113 Degersheim(CH)**

(72) Erfinder: **Herzog, Kurt**
**Sennrüti 7**
**CH-9113 Degersheim(CH)**

(74) Vertreter: **Dreiss, Hosenthien & Fuhlendorf**
**Gerokstrasse 6**
**D-7000 Stuttgart 1(DE)**

(54) **Vorrichtung zur Erleichterung des An-und Ausziehens von Handschuhen.**

(57) Zum An- und Ausziehen eines hautengen Handschuhs (H) wird der Handschuh (H) in die Oeffnung eines Gehäuses (10) gesteckt, so dass er mit seiner Manschetten-Randverstärkung (HV) auf dem Randwulst (12) aufliegt. Wird nun durch die Leitung (23) Luft aus dem Gehäuse (10) abgesaugt so dehnt sich der Handschuh (H) aus und eine Hand (A) kann mühelos hineingestossen werden. Zum Ausziehen wird wieder die behandschuhte Hand in das Gehäuse (10) gesteckt, bis die Randverstärkung (HV) am Randwulst (12) anliegt. Durch absaugen der Luft dehnt sich der Handschuh (H) aus und löst sich von der Hand, die damit ebenso mühelos wieder herausgezogen werden kann.

EP 0 347 606 A1

## Vorrichtung zur Erleichterung des An- und Ausziehens von Handschuhen

Die vorliegende Erfindung betrifft eine Vorrichtung zur Erleichterung des An- und Ausziehens von Handschuhen gemäss dem Oberbegriff des unabhängigen Patentanspruchs 1.

Die heutigen Anforderungen bezüglich der Hygiene betreffen im grossen Ausmass die Bereitstellung und die Abgabe von Lebensmitteln. Beispielsweise gibt es Fleischverkaufsläden in denen die Bedienenden das Fleisch nur mit Handschuhen anfassen dürfen. Das Tragen solcher hautengen Schutzhandschuhe während längerer Zeit ist unangenehm aber das fortwährende An- und Ausziehen, um andere Arbeiten ohne Handschuhe auszurichten ist sehr mühsam, weil beim Ausziehen die Handschuhe umgestülpt werden und dann wieder in die Gebrauchslage gekehrt werden müssen. Ein Anziehen mit feuchten Händen ist sehr beschwehrlich und das Handhaben der Handschuhe beim Umstülpen und zurückbringen beeinträchtigt die Hygiene, weil die äussere Seite mit den Händen berührt werden kann.

Es ist dementsprechend eine Aufgabe der Erfindung diesem beschwehrlichen Tun abhilfe zu schaffen.

Erfindungsgemäss wird dies durch eine Vorrichtung mit den Merkmalen im kennzeichnenden Teil des unabhängigen Patentanspruchs 1 erreicht.

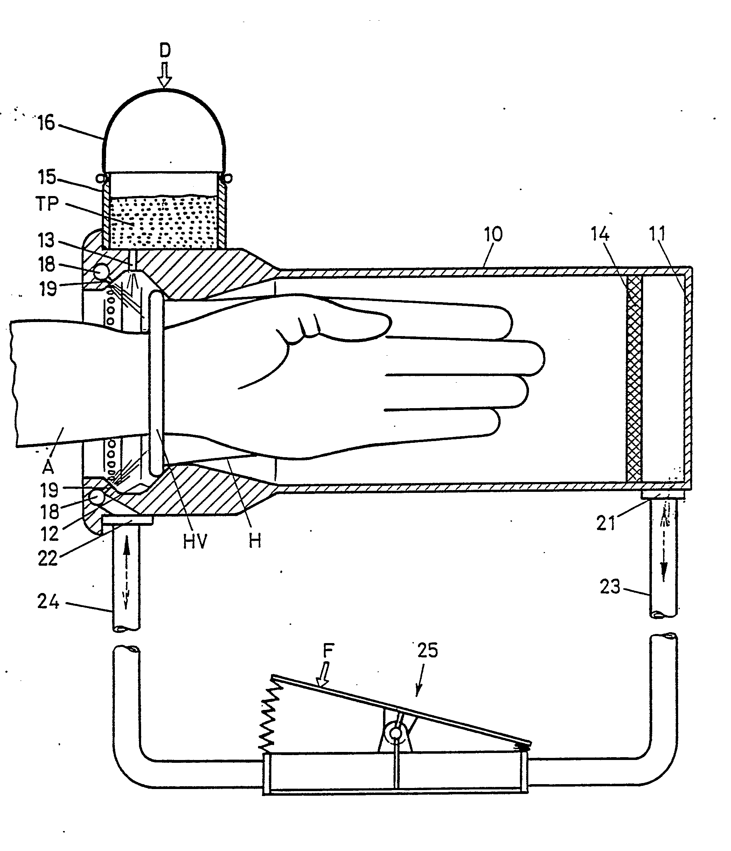

Ausführungsbeispiele werden nachfolgend beschrieben, von denen eines schematisch in der beiliegenden Zeichnung beschrieben ist.

Ein Gehäuse 10 von kreiszylindrischer oder prismatischer Form ist einerseits mit einem Boden 11 verschlossen und trägt anderseits um eine kreisrunde Oeffnung herum einen Randwulst 12.

Ein Handschuh H hat eine Randverstärkung HV in Form eines runden Wulstes. Mit diesem Wulst liegt der Handschuh H luftdicht am Randwulst 12 des Gehäuses 10 an. Im Gebiet des Bodens 11 oder im Boden 11 selbst sind Anschlüsse von denen in der Zeichnung der Anschluss 21 gezeichnet ist. Durch absaugen der Luft aus dem Gehäuse 10 dehnt sich der Handschuh H aus und der Bedienende kann seine Hand A leicht und ohne Mühe hineinstecken. Durch nachfolgenden Druckausgleich umschliesst der Handschuh H die Hand A und wird mit der Hand aus dem Gehäuse 10 herausgezogen. Zum Ausziehen steckt der Bedienende die handschuhbewehrte Hand A in das Gehäuse, bis dessen Randverstärkung HV am Randwulst 12 des Gehäuses 10 anliegt, schaltet die Luftabsaugvorrichtung ein, so dass sich der Handschuh H unter dem gebildeten Unterdruck ausdehnt, und die Hand A kann wieder mühelos herausgezogen werden.

Um das hineinschlüpfen noch weiter zu erleichtern trägt das Gehäuse 10 einen Behälter 15 für Talcum-Puder TP. Der Behälter 15 ist mit einem Gummiball 16 versehen und ein Durchgang 13 durch den Randwulst 12 lässt bei Betätigung des Gummiballs 16 in Richtung des Pfeils D eine Menge des Talcum-Puder herausfallen. Mit einer Ringleitung 18 mit auf dem Umfang verteilt angeordneten Düsen 19 gestattet, dass mit Pressluft aus einer Leitung am Anschluss 22 der Talcum-Puder in den Handschuh A geblasen wird.

Ein Filter 14 vor dem Anschluss 21 bewirkt, dass keine festen Gegenstände, die möglicherweise noch am Handschuh H anhaften durch den Anschluss 21 in die Evakuierleitung gelangen können.

Es ist dementsprechend möglich, mit Leitungen an mehreren Anschlüssen Druckluft einzubringen oder den Innenraum des Gehäuses 10 zu evakuieren.

In der Zeichnung ist eine fussbetätigte Pumpe 25 angedeutet, die mit einem Druck in Richtung des Pfeiles F z.B. rechts Luft ansaugt und links Luft ausstösst. Die entsprechenden Verbindungsleitungen 23, 24 zu den Anschlüssen 21, 22 können flexible oder starre Leitungen sein.

Mit einer derartigen Vorrichtung kann gleichzeitig Luft zwecks ausdehnens des Handschuhs abgesaugt und Luft zur Unterstützung des Dehnvorgangs hineingeblasen werden. Beim Ausziehen wird die Pumpe 25 ein zweites Mal mit Druck in Richtung des Pfeils F betätigt und der Handschuh H dehnt sich aus, so dass die Hand A ohne Mühe herausgenommen werden kann. Die Düsen 19 geben auch wieder einen Luftstrahl ab, aber wenn der Ball 16 nicht betätigt wird, kann auch kein Talcum-Puder verstäubt werden. Im Gegenteil, an der Hand noch anhaftender Puder kann dadurch wieder in den Handschuh zurück befördert werden.

## Ansprüche

1. Vorrichtung zur Erleichterung des An- und Ausziehens von Handschuhen, insbesondere von hautengen Schutzhandschuhen, gekennzeichnet durch ein Gehäuse (10) für die bequeme Aufnahme einer menschlichen, handschuhbewehrten Hand (A) mit einerseits einer von einem Anlageflansch (12) für die Auflage des Randwulstes (HV) eines Handschuhs (H) umgebenen Oeffnung und anderseits einer mit Leitungen (21) für die Zufuhr und/oder Entnahme von Luft durchstossenen Bodenpartie (11).

2. Vorrichtung nach Patentanspruch 1, dadurch gekennzeichnet, dass die Oeffnung vor dem Anlageflansch (12) einen Durchgang (13) durch die Wand des Gehäuses (10) aufweist, die eine Verbindung zu einem Talcum-Behälter (16) ausserhalb des Gehäuses (10) schafft.

3. Vorrichtung nach Patentanspruch 1 oder Patentanspruch 2, dadurch gekennzeichnet, dass der Talcum-Behälter (15) mit einem Gummiball (16) zur Abgabe einer Menge des Talcum-Puders (TP) aus dem Behälter (15) versehen ist.

4. Vorrichtung nach Patentanspruch 3, gekennzeichnet durch eine Ringleitung (18) im Randbereich des Gehäuses (10) mit auf dem Umfang verteilt angeordneten Düsen (19) zum Einblasen von aus dem Behälter (15) abgegebenen Talcum-Puders (TP).

5. Vorrichtung nach einem der Patentansprüche 1 bis 4, dadurch gekennzeichnet, dass im Gehäuse (10) bei der Bodenpartie (11) eine Filteranordnung (14) vorhanden ist, um zu verhindern, dass Fremdkörper durch die Leitungen (23) abgesaugt werden.

6. Vorrichtung nach einem der Patentansprüche 1 bis 5, gekennzeichnet durch eine Fusstritt-Pumpe (25) die einerseits mit einer Verbindungsleitung (23) zur Bodenpartie (11) des Gehäuses (10) und anderseits mit einer Verbindungsleitung (24) zur Ringleitung (18) verbunden ist.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | US-A-1 996 377 (HINCHEN)<br>* Spalte 2, Zeile 12 - Spalte 3, Zeile 9; Figur 1 *<br>--- | 1 | A 61 B 19/04 |
| X | US-A-1 938 685 (BREULS)<br>* Seite 1, Zeilen 46-54,86-103; Figur 1 *<br>----- | 1 | |
| | | | **RECHERCHIERTE SACHGEBIETE (Int. Cl.4)**<br><br>A 61 B |

Der vorliegende Recherchenbericht wurde für alle Patentansprüche erstellt

| Recherchenort | Abschlußdatum der Recherche | Prüfer |
|---|---|---|
| DEN HAAG | 15-09-1989 | MOERS R.J. |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus andern Gründen angeführtes Dokument
........................................................................
& : Mitglied der gleichen Patentfamilie, übereinstimmendes
    Dokument

EPO FORM 1503 03.82 (P0403)